# EUROPEAN PATENT SPECIFICATION

(11) **EP 2 467 100 B1**
(45) Date of publication and mention of the grant of the patent: **11.11.2015**
(21) Application number: 09812489.4
(22) Date of filing: 20.08.2009
(51) Int. Cl.: B01F 11/02, A61B 17/88, B01F 13/00, B01F 15/00

(54) **BONE CEMENT MIXER**
KNOCHENZEMENTMISCHER
MELANGEUR DE CIMENT OSSEUX

(43) Date of publication of application: 27.06.2012
(73) Proprietor: Tecres S.P.A., 37066 Sommacampagna (VR) (IT)
(72) Inventor: FACCIOLI, Giovanni, I-37066 Sommacampagna (Verona) (IT); SOFFIATTI, Renzo, I-37066 Sommacampagna (Verona) (IT)
(74) Representative: Feltrinelli, Secondo Andrea
(86) International application number: PCT/IB2009/053675
(87) International publication number: WO 2011/021072

(56) References cited:
- EP-A2- 1 466 572
- DE-U1- 20 216 631
- JP-A- 10 314 181
- SU-A1- 916 332
- SU-A1- 1 047 700
- US-A- 5 088 830
- US-A1- 2002 118 595

## Description

The invention refers to a bone cement mixer.

In surgery, bone cement is very often used for the reconstruction of bones.

It is normally prepared through two components, one liquid (monomer) and one in powder (polymer), kept separate from one another right until they are mixed at which point they bond together. The mixing is carried out when the worker pours the liquid component into the container with the powder to form a cement-like mixture.

It is preferable to mix the two components in closed containers, in order to avoid diffusion of vapours. These containers are usually equipped with a mixer member, usually a piston or a spatula which can be actuated manually. The cement is then transferred and injected onto the bone through a syringe.

The document n. JP 10314181 discloses a mixing device there the mixing of the bone cement is obtained by manual or machine oscillation of the whole device.

Documents n. SU 1047700 and SU 916332 disclose devices for the activation of cement using vibrators.

The document EP 1466572 discloses a packaging, mixing and injecting device for bone cement where a mixing ball is inserted inside the mixing chamber.

US5088830A teaches a mixer including a container part and a lid connected one with the other, thereby delimiting an inner space. Moreover, the mixer is clamped between two claws of a vibratory mixing device.

It is a trend for surgeons to search for ever more dense cement, because they are more easily applied whilst having a lower tendency to leak inside the body. However, having a higher density requires more strength while mixing, and therefore there is the parallel problem of suitably mixing the two cement components without excessive strain.

The object of the invention is that of making the aforementioned mixing of the cement in medical devices easier obtaining it through "knocking or vibration" applied to the walls of the mixer.

Such an object is obtained with a mixer according to claim 1.

The generating and transmitting means can be made in many ways.

An electric energy vibration generator can be used, like for example an eccentric fitted onto an electric motor in abutment against a wall of the mixing chamber. Power can be supplied, for example, through batteries integrated in the mixer. Otherwise, the shaking movement which is given to the mixer by the worker during the mixing could be exploited. In this case it is advantageous to make the means for generating and transmitting vibrations having a mass free to oscillate (knocker) and knock against an outer side of at least one wall of the mixing chamber.

The free mass can be a sphere or in general a body fixedly connected to the mixer, for example, by a cable. Or the mass can be contained inside a closed chamber which shares a wall with the mixing chamber, thus improving its integration with the mixer and making it more compact.

In order to increase the effectiveness of the knocking mass, the shared wall between said closed chamber and said mixing chamber is equipped with thinner portions to favour a sussultatory movement of the shared wall itself. The rigidity of the shared wall is thus reduced increasing the bending capacity thereof and further moving the forming cement.

The invention also solves the coupling problem of the mixer with the device used to inject the cement into the bone, usually a syringe or a press dispenser.

The mixer should have a structure able to prevent contamination of the cement from the environment and make the application and use operations in the operating room easier.

For this purpose, the invention providesng the mixer with a luer fitting through which the cement can exit, the luer fitting being easily coupled with a syringe and/or dosage tubes. Preferably the mixer comprises a luer cap for the luer fitting.

Further characteristics and advantages of the invention shall become clearer from the description given as an example of a mixer, together with the attached drawings in which:
figure 1 shows a side view of a mixer according to the invention;
figure 2 shows a section view of the mixer of fig. 1 according to the plane A-A of fig. 1;
figure 3 shows a side view of a component of the mixer of fig. 1;
figure 4 shows a section view of the component of fig. 3 according to the plane B-B of fig. 3;
figure 5 shows a side view of a second mixer according to the invention;
figure 6 shows a side view of a second mixer according to the invention.

A mixer according to the invention is indicated with reference numeral 10.

Said mixer comprises a cylindrical case 12 which extends along an axis X and which defines a mixing chamber 20 inside of it, and another open chamber 40 adjacent to the first one and with which it shares a common wall 30.

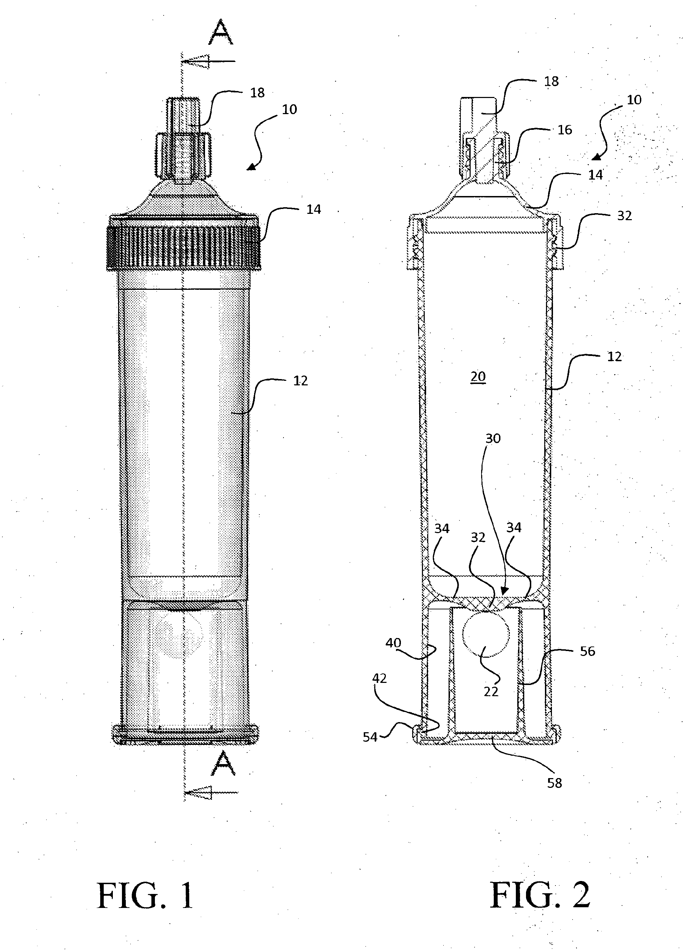

The mixing chamber 20 is open towards the outside and at the corresponding end of the mixer 10, on its outer side surface, there is a thread 32 onto which a lid 14 can be screwed to close the chamber, forming one of its walls (see fig. 2). Once the components to be mixed have been deposited in the chamber 20, the cap 14 is applied and closed. The lid 14 is tapered and has a luer fitting opening 16 at its apex, closed by a luer cap 18.

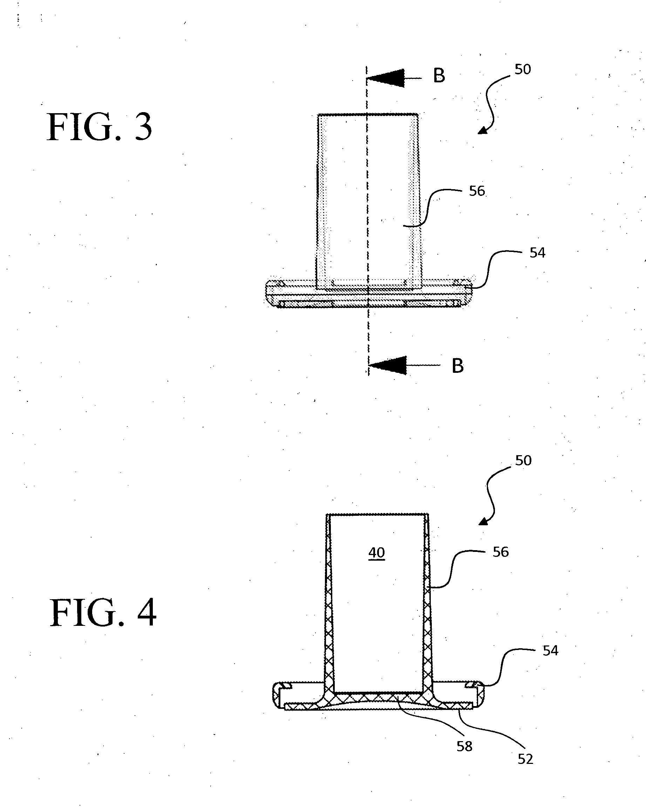

A closing element 50 can be inserted snapping into the chamber 40 (see figs. 3 and 4). The closing element comprises a circular base 52 and a hollow cylindrical central body 56 open at one end and closed at the bottom 58. The base 52 has a peripheral lip 54 suitable for snapping onto an edge in relief 42 on the outside of the case 12. Inside the body 56 there is a small sphere 22, smaller in size, which is free to move inside of the body and to knock against its walls.

The chamber 40 has an oblong shape and a depth almost equal to the height of the body 56, so that the second one can be inserted into the first (fig. 3). It should be seen that the small sphere 22 can substantially move with a good clearance from and towards the wall 30 (along the axis X) staying within the body 56.

The section of the wall 30 is not even. It has a central portion 32 which is thicker and an annular portion 34 which is thinner. In this way the ability of the portion 32 to oscillate with respect to the case 12 is increased.

The operation of the mixer 10 is as follows. The element 50 is applied to the mixer 10 and the components to be mixed are introduced into the chamber 20, after closing it with the lid 14. Then the mixer 10 should be held in hand and shaken. Consequently, the small sphere 22 is set in motion inside the chamber 40 and repeatedly knocks against the wall 30. The mixing of the components is obtained thanks to manual shaking, and a further mixing effect is developed through the vibrations generated by the impact of the sphere 22 against the wall 30. Even though the cement has high density, such vibrations are unexpectedly able to improve the mixing.

It should also be noted that the funnel-shaped hermetically sealing lid 14 ends with a luer fitting 16, which allows a syringe to be connected to it and thus directly aspirate the mixed cement. This means there is an optimal asepticity of the cement. The luer cap 18 further improves the characteristics of adaptability and simplicity of use of the mixer 10.

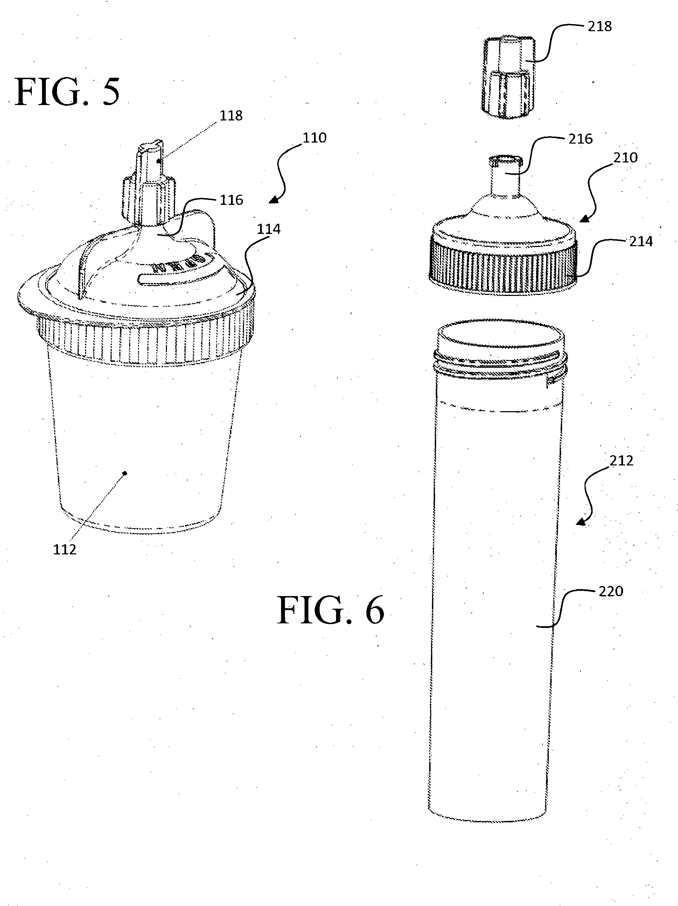

The solution with luer fitting can be implemented to other mixers as well, with knocking mass. Figs. 5 and 6 show some examples, the first one is of a cup mixer 110 which comprises a cylindrical case 112 and which defines a mixing chamber inside of it. Such a mixing chamber is open towards the outside and it is closed by a tapered lid 114 which has a luer fitting 116 at its apex, and it is closed by a luer cap 118.

The second mixer 210 comprises a cylindrical case 212 and it defines a mixing chamber 220 inside of it Such a mixing chamber is open towards the outside and it is closed by a tapered lid 214 which has a luer attachment 216 at its apex and it is closed by a luer cap 218.

## Claims

1. Mixer (10) for obtaining bone cement for medical applications comprising
a mixing chamber (20) wherein the mixing of at least two components which form the cement can be carried out, the chamber (20) being defined by walls of the mixer (10),
**characterized in that** it comprises means for generating and transmitting vibrations at the outer side of at least one wall of the mixing chamber (20), wherein the means for generating and transmitting vibrations comprise a mass (22) free to oscillate and knock against an outer side of at least one wall of the mixing chamber (20).

2. Mixer according to claim 1, wherein said mass (22) is contained inside a closed chamber (40) which shares a common wall (30) with the mixing chamber (20).

3. Mixer according to claim 2, comprising a main body which defines the mixing chamber (20) and an element able to be permanently coupled with the main body, the element having a cavity, in which said mass (22) can move, that has an opening, from which said mass can exit, which, once coupled, faces towards the common wall (30).

4. Mixer according to claim 2 or 3, wherein the common wall (30) between said closed chamber (40) and said mixing chamber (20) has thinner portions (34) to favour a sussultatory movement of the common wall (30) itself.

5. Mixer according to anyone of the previous claims, comprising a luer fitting (16) through which the cement can exit.

6. Mixer according to claim 5, comprising a luer cap (18) for the luer fitting (16).

7. Mixer according to anyone of the previous claim when depending upon claim 2, comprising a cylindrical case (12) which extends along an axis (X) and which defines said mixing chamber (20) inside of it, and defines said closed chamber (40) adjacent to said mixing chamber (20), and comprising a closing element (50) snap-inserteable into said closed chamber (40), said closing element (50) comprising a circular base (52) and a hollow cylindrical central body (56) open at one end and closed at the bottom (58), said circular base (52) having a peripheral lip (54) suitable for snapping onto an edge in relief (42) on the outside of said cylindrical case (12), said mass (22) being inside said hollow cylindrical central body (56).

8. Mixer according to claim 7, wherein said chamber (40) has an oblong shape and a depth almost equal to the height of said hollow cylindrical central body (56), so that said hollow cylindrical central body (56) is inserteable into said chamber (40).

9. Mixer according to anyone of the previous claim, wherein said mixing chamber (20) is opened towards the outside and at the corresponding end of the mixer (10), on the outer side surface thereof, there is a thread (32), and wherein it comprises a lid (14) screawable onto said thread (32) to close said mixing chamber (20) and forming one of its walls.

10. Mixer according to anyone of the previous claim, wherein said mass comprises a small sphere (22).

11. Method of operating a mixer as claimed in claims 7 and 9 or in claims 8 and 9, comprising the following steps:
- closing said mixing chamber (20) with said lid (14) and introducing the components to be mixed into said mixing chamber (20);
- holding in hand and manually shaking said mixer (10), thereby setting in motion said mass (22) inside said closed chamber (40), so that said mass (22) repeatedly knocks against said common wall (30).

## Patentansprüche

1. Mischer (10) zur Herstellung von Knochenzement für medizinische Anwendungen, umfassend:
eine Mischkammer (20), in der das Mischen von mindestens zwei Komponenten, die den Zement bilden, erfolgen kann, wobei die Kammer (20) von Wänden des Mischers (10) definiert wird,
**dadurch gekennzeichnet, dass** er Mittel zum Erzeugen und Übertragen von Schwingungen auf die Außenseite von mindestens einer Wand der Mischkammer (20) umfasst, wobei die Mittel zum Erzeugen und Übertragen von Schwingungen eine Masse (22) umfassen, die frei schwingen und gegen eine Außenseite von mindestens einer Wand der Mischkammer (20) stoßen kann.

2. Mischer nach Anspruch 1, wobei die Masse (22) in einer geschlossenen Kammer (40) enthalten ist, die eine gemeinsame Wand (30) mit der Mischkammer (20) teilt.

3. Mischer nach Anspruch 2, umfassend einen Hauptkörper, der die Mischkammer (20) definiert, und ein Element, das dauerhaft mit dem Hauptkörper verbunden sein kann, wobei dieses Element einen Hohlraum, in dem sich die Masse (22) bewegen kann, aufweist, der eine Öffnung, durch die die Masse austreten kann, aufweist, die, wenn verbunden, der gemeinsamen Wand (30) zugewandt ist.

4. Mischer nach Anspruch 2 oder 3, wobei die gemeinsame Wand (30) zwischen der geschlossenen Kammer (40) und der Mischkammer (20) dünnere Abschnitte (34) aufweist, um eine auf und ab schwingende Bewegung der gemeinsamen Wand (30) selbst zu begünstigen.

5. Mischer nach einem der vorhergehenden Ansprüche, umfassend einen Luer-Anschluss (16), durch den der Zement austreten kann.

6. Mischer nach Anspruch 5, umfassend eine Luer-Verschlusskappe (18) für den Luer-Anschluss (16).

7. Mischer nach einem der vorhergehenden Ansprüche, wenn abhängig von Anspruch 2, umfassend ein zylindrisches Gehäuse (12), das sich entlang einer Achse (X) erstreckt und die Mischkammer (20) in ihm definiert und das die an diese Mischkammer (20) angrenzende geschlossene Kammer (40) definiert, und umfassend ein Verschlusselement (50), das einrastend in die geschlossene Kammer (40) einsetzbar ist, wobei dieses Verschlusselement (50) ein kreisrundes Unterteil (52) und einen hohlzylindrischen Zentralkörper (56) umfasst, der an einem Ende offen und am Boden (58) geschlossen ist, wobei dieses kreisrunde Unterteil (52) eine Umfangslippe (54) aufweist, die auf einem erhabenen Rand (42) auf der Außenseite des zylindrischen Gehäuses (12) eingerastet werden kann, wobei sich die Masse (22) innerhalb des hohlzylindrischen Zentralkörpers (56) befindet.

8. Mischer nach Anspruch 7, wobei die Kammer (40) eine längliche Form und eine Tiefe, die fast gleich der Höhe des hohlzylindrischen Zentralkörpers (56) ist, aufweist, so dass der hohlzylindrische Zentralkörper (56) in diese Kammer (40) eingefügt werden kann.

9. Mischer nach einem der vorhergehenden Ansprüche, wobei die Mischkammer (20) nach außen offen ist und sich an dem entsprechenden Ende des Mischers (10), auf der äußeren Seitenfläche desselben, ein Gewinde (32) befindet, und wobei er einen Deckel (14) umfasst, der auf dieses Gewinde (32) geschraubt werden kann, um die Mischkammer (20) zu verschließen und eine ihrer Wände zu bilden.

10. Mischer nach einem der vorhergehenden Ansprüche, wobei die Masse eine kleine Kugel (22) umfasst.

11. Verfahren zum Betätigen eines Mischers nach den Ansprüchen 7 und 9 oder nach den Ansprüchen 8 und 9, das die folgenden Schritte umfasst:
- Verschließen der Mischkammer (20) mit dem Deckel (14) und Einbringen der zu mischenden Komponenten in die Mischkammer (20);
- Inderhandhalten und manuelles Schütteln des Mischers (10) und dadurch Inbewegungversetzen der Masse (22) in der geschlossenen Kammer (40) derart, dass diese Masse (22) wiederholt gegen die gemeinsame Wand (30) stößt.

## Revendications

1. Mélangeur (10) pour obtenir un ciment osseux pour applications médicales comprenant
une chambre de mélange (20) dans laquelle le mélange d'au moins deux composants qui forment le ciment peut être exécuté, la chambre (20) étant définie par des parois du mélangeur (10),
**caractérisé en ce qu'**il comprend des moyens pour générer et transmettre des vibrations au niveau du côté extérieur d'au moins une paroi de la chambre de mélange (20), dans lequel les moyens pour générer et transmettre des vibrations comprennent une masse (22) libre d'osciller et de cogner contre un côté extérieur d'au moins une paroi de la chambre de mélange (20).

2. Mélangeur selon la revendication 1, dans lequel ladite masse (22) est contenue à l'intérieur de la chambre fermée (40) qui partage une paroi commune (30) avec la chambre de mélange (20).

3. Mélangeur selon la revendication 2, comprenant un corps principal qui définit la chambre de mélange (20) et un élément adapté pour être accouplé de manière permanente avec le corps principal, l'élément ayant une cavité, dans lequel ladite masse (22) peut se déplacer, qui a une ouverture, par laquelle ladite masse peut sortir, qui, une fois accouplé, fait face vers la paroi commune (30).

4. Mélangeur selon la revendication 2 ou 3, dans lequel la paroi commune (30) entre ladite chambre fermée (40) et ladite chambre de mélange (20) a des portions plus minces (34) pour favoriser un mouvement de type à sursauts de la paroi commune (30) elle-même.

5. Mélangeur selon l'une quelconque des revendications précédentes, comprenant un raccord Luer (16) à travers lequel le ciment peut sortir.

6. Mélangeur selon la revendication 5, comprenant un capuchon Luer (18) pour le raccord Luer (16).

7. Mélangeur selon l'une quelconque des revendications précédentes quand elles dépendant de la revendication 2, comprenant un boîtier cylindrique (12) qui s'étend suivant un axe (X) et qui définit à l'intérieur ladite chambre de mélange (20) et définit ladite chambre fermée (40) adjacente à ladite chambre de mélange (20), et comprenant un élément de fermeture (50) pouvant être inséré par encliquetage dans ladite chambre fermée (40), ledit élément de fermeture (50) comprenant une base circulaire (52) et un corps central cylindrique creux (56) ouvert à une extrémité et fermé au niveau du fond (58), ladite base circulaire (52) ayant une lèvre périphérique (54) adapté pour s'encliqueter sur un bord en relief (42) sur l'extérieur dudit boîtier cylindrique (12), ladite masse (22) étant à l'intérieur dudit corps central cylindrique creux (56).

8. Mélangeur selon la revendication 7, dans lequel ladite chambre (40) a un profil oblong et une profondeur pratiquement égale à la hauteur dudit corps central cylindrique creux (56), de manière que ledit corps central cylindrique creux (56) puisse être inséré dans ladite chambre (40).

9. Mélangeur selon l'une quelconque des revendications précédentes, dans lequel ladite chambre de mélange (20) est ouverte vers l'extérieur et au niveau de l'extrémité correspondante du mélangeur (10), un filetage (32) est présent sur sa surface latérale extérieure, et dans lequel il comprend un couvercle (14) pouvant être vissé sur ledit filetage (32) pour fermer ladite chambre de mélange (20) et former une de ses parois.

10. Mélangeur selon l'une quelconque des revendications précédentes, dans lequel ladite masse comprend une petite sphère (22).

11. Procédé de fonctionnement d'un mélangeur selon les revendications 7 et 9 ou les revendications 8 et 9, comprenant les étapes suivantes :
- la fermeture de ladite chambre de mélange (20) avec ledit couvercle (14) et l'introduction des composants à mélanger dans ladite chambre de mélange (20) ;
- le fait de prendre en main et agiter manuellement ledit mélangeur (10), en mettant ainsi en mouvement ladite masse (22) à l'intérieur de ladite chambre fermée (40), de manière que ladite masse (22) cogne à maintes reprises contre ladite paroi commune (30).
